Europäisches Patentamt

European Patent Office

Office européen des brevets

(11) Veröffentlichungsnummer: **0 589 384 A2**

## (12) EUROPÄISCHE PATENTANMELDUNG

(21) Anmeldenummer: **93115084.1**

(22) Anmeldetag: **20.09.93**

(51) Int. Cl.⁵: **G01N 37/00**, G01N 33/00, G01N 33/487

(30) Priorität: **25.09.92 DE 4232141**

(43) Veröffentlichungstag der Anmeldung:
**30.03.94 Patentblatt 94/13**

(84) Benannte Vertragsstaaten:
**CH DE FR LI NL**

(71) Anmelder: **Forschungszentrum Jülich GmbH**
**Wilhelm-Johnen-Strasse**
**D-52425 Jülich(DE)**

(72) Erfinder: **Freyer, Stephan**
**Oberer Röderweg 51**
**D-67434 Neustadt a.d. Weinstrasse(DE)**
Erfinder: **Wandrey, Christian, Prof.**
**Wolfshovener Strasse 139**
**D-52428 Jülich(DE)**

(54) **Verfahren zur experimentellen Ermittlung der Parameter für optimale Ergebnisse.**

(57) Der experimentelle Aufwand zur Ermittlung der Daten für die Optimalbedingungen eines von einer Vielzahl von Einflußgrößen abhängenden Prozesses wird durch eine programmgesteuerte Versuchsstrategie stark vermindert, indem ausgehend von Zufallswerten für die einzelnen Parameter und den damit erzielten Ergebnissen (erste Versuchsgruppe) eine dem Evolutions-Algorithmus folgende Auswahl der zweiten und folgenden Versuchsgruppen von Parameterwerten mit Wichtung am Ergebnismittelwert getroffen wird, mit zusätzlicher Zufallsabwandlung, wodurch sichergestellt wird, daß vorhandene Optima nicht "übersehen" werden. Bei der Realisierung erfolgt eine Codierung der Parameterwerte in Form von Bitstrings, deren Anzahl der Versuchszahl einer Versuchsgruppe entspricht und die in eine der Zahl der Parameter entsprechende Anzahl von Segmenten mit einer der gewünschten Rasterung entsprechenden Bitzahl unterteilt sind. Nach jeder Versuchsgruppe erfolgt zur Festlegung der Daten der nächsten eine am Ergebnis gewichtete Zufallsauswahl unter abgestufter Anwendung von Cross-over-, Inversions- und Mutationsauswahlprinzipien.

EP 0 589 384 A2

Die Erfindung bezieht sich auf ein iteratives Verfahren zur experimentellen Ermittelung der Parameterwerte für ein optimales Prozeßergebnis innerhalb von vorgegebenen Bereichsgrenzen für eine Vielzahl von Parametern nicht bekannter Interdependanz und Ergebnis-Relevanz durch Aufeinanderfolge von Versuchsreihen mit jeweils innerhalb der Bereichsgrenzen abgewandelten Parametervorgaben bzw. auf ein Verfahren zur parameter-abhängigen Ergibnis-Kartographierung.

Dabei sollen insbesondere innerhalb komplexer Systeme mit nicht mehr überschaubaren oder unbekannten Abhängigkeiten OptimaParameter mit möglichst geringem Aufwand an praktischen Versuchen ermittelt werden. Es können ein oder oder mehrere Ziele angestrebt werden. Als Beispiel für ein erfindungsgemäß zu lösendes Problem ist etwa die Ermittlung der Nährstoff-Zusammensetzung eines Fermentationsmediums zur Bildung eines bestimmten Produktes mit optimaler Raumzeitausbeute zu nennen, ggf. unter Beachtung minimaler Herstellungskosten.

Biotechnologische Prozesse sind exemplarische Beispiele für schwer modellierbare und experimentell relativ gut beherrschbare Systeme. Eine weitere Eigenschaft sind die stark verrauschten Daten der Experimente.

Eine grundlegende Rolle bei der Entwicklung biotechnologischer Prozesse spielt nach der Stammentwicklung (Isolierung, Mutation, genetischer Modifikation und Selektion), die Optimierung der Reaktionsbedingungen.

Neben pH-Wert und Temperatur ist dabei die qualitative und quantitative Zusammensetzung des Nährmediums von entscheidender Bedeutung.

Die für jeden Stamm individuelle optimale Nährmedien-Zusammensetzung beinhaltet ein hohes, wenig genutztes Optimierungspotential und ist ein wichtiger ökonomischer Faktor eines Fermentationsprozesses.

Im allgemeinen werden neben der aufwendigen und wenig effizienten Variation nur eines Medienbestandteils ("one-faktor-at-a-time" Methode) Methoden der statistischen Versuchsplanung eingesetzt, um die Zahl der Versuche zu verringern. In vielen Bereichen, beispielsweise in der Prozeßentwicklung bei chemischen Verfahren oder der Qualitätssicherung, wird sie angewandt.

Problematisch für ihren Einsatz in der Biotechnologie sind:

exponentielle Zunahme der Versuche mit der Parameter-Zahl,

Annahme eines linearen Modells,

Empfindlichkeit gegenüber Meßrauschen.

Bei fünf Parametern und zwei Konzentrations-Stufen benötigt man $2^5$ = 32 Experimente, für zehn Parameter schon $2^{10}$ = 1024 Versuche. Will man eine feinere Rasterung der Parametereinstellungen vornehmen, z.B. fünf Stufen bei fünf Parametern, benötigt man $5^5$ = 3125 Experimente. Der hohen Versuchsanzahl kann man durch Verkleinerung der Versuchspläne entgegentreten, was wiederum zu Vernachlässigungen der Wechselwirkungen zwischen den einzelnen Parametern führt. Die Annahme linearer Modelle ist gerade bei den nicht linearen biologischen Systemen eine ungünstige Vereinfachung.

Das biologischen Systemen eigene Problem der schlechten Reproduzierbarkeit der Daten unter nicht standardisierten Bedingungen wirft ein weiteres Problem auf. Mit statistischen Methoden kann man das Rauschen erfassen und so eine Aussage über die Qualität der Daten bekommen. Letztendlich wird man aber bei diesen Optimierverfahren Messungen mit prozentualen Standardabweichungen oberhalb von 5 - 10 % wiederholen müssen.

Auch andere Optimierverfahren, wie z.B. die Rosenbrock- oder die Simplex-Methode, scheitern in komplexen, hochparametrigen Systemen aufgrund der bei ihrer Entwicklung zugrundegelegten Vorstellungen der zu untersuchenden Systeme (siehe Schwefel H.-P. (1977), Numerische Optimierung von Computer-Modellen mittels der Evolutionsstrategie, Birkhäuser Verlag, Basel und Stuttgart).

Ziel der Erfindung ist daher eine Versuchsstrategie, die mit einer möglichst geringen Anzahl von Versuchen auskommt und trotzdem eine weitgehende Sicherheit bietet, daß Optima "nicht übersehen" werden.

Das zu diesem Zweck entwickelte erfindungsgemäße Verfahren der eingangs genannten Art ist dadurch gekennzeichnet, daß die Parametervorgaben jeder Versuchsreihe - ausgehend von den Ergebniswerten einer ersten, durch Zufallswerte der Parameter festgelegten Versuchsreihe - durch Anwendung einer dem genetischen Evolutions-Algorithmus (nach Holland) folgenden Optimierungsstrategie unter Zugrundelegung der Ergebniswerte der jeweils vorangehenden Versuchsreihe festgelegt werden.

Eine solche Optimierungsstrategie für die Auswahl von Parameterwerten für experimentell durchzuführende Versuchsreihen bei der am Ergebnis einer Versuchsreihe gewichtete, zufallsmodifizierte Zahlenwerte Basis für die Parametervorgaben der nächstfolgenden Versuchsreihe sind, ist nützlich bei der experimentellen Suche nach Optimierungen innerhalb von Systemen mit einer sehr großen Vielzahl von Einflußgrößen (Parametern), deren Abhängigkeit untereinander und Einwirkung auf das zu erzielende Ergebnis weitgehend

EP 0 589 384 A2

unbekannt sind oder auch wenn mehrere Zielsetzungen gleichzeitig zu beachten sind, um mit erheblich vermindertem Versuchsaufwand zu Optimierungen zu gelangen. Als "Prozeßergebnis" wird dabei ein durch direkte Einflußnahme auf die praktischen Voraussetzungen veränderbares Ergebnis verstanden.

Die erfindungsgemäße Versuchsplanung kann einfach unter Anwendung des Optimierungsprinzips nach Holland durchgeführt werden, selbstverständlich sind jedoch auch die zwischenzeitlich in großer Vielfalt entwickelten Abwandlungen desselben einsetzbar, wie sie zusammenfassend von D.E.Goldberg in "Genetic Algorithms in Search Optimization and Machine Learning", Addison Wesley Publishing Company, Inc. Reading, Massachusetts, 1989 sowie von Lawrence Davis "Genetic Algorithm Tutorial" in Handbook of Genetic Algorithms (ed.L.Davis), Van Nostrand Reinhold Verlag New York, 1991 dargestellt sind und insbesondere die Abwandlung der Einzelschritte z.B. in Form von Mehrpunkt-crossover, oder unterschiedlicher Selektionsmechanismen oder auch die Angabe von "Hybriden Algorithmen" betreffen.

Eine zweckmäßige Realisierung des erfindungsgemäßen Verfahrens ergibt sich dadurch, daß

Ia) eine der beliebig wählbaren Anzahl der Versuche der primären Versuchsreihe entsprechende Anzahl von Bitstrings mit einer der Anzahl der Parameter entsprechenden Anzahl von Segmenten mit jeweils der gewünschten Rasterung entsprechenden Bitzahl durch Zufallskodierung gebildet wird,

Ib) deren numerisches Ergebnis multipliziert mit dem Quotienten aus Abtastbereich durch maximalen decodierten Bitwert zum Parameter-Minimalwert addiert und ggf. aufgerundet je Segment die Realwerte der einzelnen Versuchsparameter der primären Versuchsreihe ergibt, daß

II) die Versuche der primären Versuchsreihe mit den so ermittelten Parametern durchgeführt werden unter Erzielung einer Gruppe von Ergebnissen und daß

III) aus den erhaltenen Ergebnissen der Ergebnismittelwert gebildet wird, der einen Wichtungsschlüssel (aus Einzelergebnis der Versuchsreihe / Ergebnismittelwert der Versuchsreihe) liefert für die Zufallsauswahl einer intermediären Bitstring-Folge, die nacheinander mit abgestufter Wahrscheinlichkeit einem Cross-over (a), der Inversion (b) und der Mutation (c) unterworfen wird, wobei

a) innerhalb der Bitstring-Folge eine Zufallspaarung über alle Bitstrings vorgenommen wird und bei den so gebildeten Bitstringpaaren ein Austausch der einer zufällig gewählten Position folgenden Bits untereinander vorgenommen wird, worauf

b) hinter einer oder zwischen mehreren zufällen Positionen der mit gegenüber (a) mit geminderter Wahrscheinlichkeit ausgewählten Bitstrings eine Umkehr der Bit-Reihenfolge unter Beibehaltung ihrer Parameterzugehörigkeit und Platznummer stattfindet und schließlich

c) mit wiederum verminderter Wahrscheinlichkeit ein Zufallsaustausch einzelner Bits vorgesehen wird, daß die auf diese Weise erhaltene Bitstringfolge der anschließenden Versuchsreihe gemäß Ib in Realwerte umgesetzt wird, wobei ggf. der gemäß Wichtung beste Bitstring unverändert in die nächste Bitstringfolge eingereiht wird; und daß nach Durchführung der Versuche entsprechend der so festgelegten Versuchsvorschriften ggf. wiederkehrend erneut wie unter III verfahren wird.

Bei der vorstehenden Definition der Erfindung wurden zur Erleichterung des Verständnisses "Bits" und "Bitstrings" für Strukturelemente und Strukturelementketten eingesetzt, die selbstverständlich auch durch jedes andere Zahlen- oder Buchstabensystem realisiert werden können. Auch solche Realisierungen sollen hier miterfaßt werden.

Die Genetischen Algorithmen (GA) wurden von J.H. Holland 1975 publiziert ("Adaption in natural and artificial systems", The University of Michigan Press Ann Arbor, Michigan). Bei den hier eingesetzten GA wurden auch Methoden integriert, die von anderen Autoren stammen. Diese Techniken wurden ursprünglich mit dem Ziel erarbeitet, eine Simulation der Entwicklung von Systemen mit komplexen Abhängigkeiten auf einem Rechner zu ermöglichen. Beispiele sind:

Funktionenoptimierung;

Bilderkennung;

Spieltheorie;

Simulation der Evolution;

Parameteridentifikation;

Auslegen von Schaltkreisen;

Optimierung von Gaspipelines.

Erfindungsgemäß wird nun dieser bislang als Rechenmodell für EDVisierte Abschätzungen bzw. Berechnungen von Entwicklungen, Zusammenhängen oder Verhaltensweisen innerhalb komplizierter Systeme benutzte Algorithmus für die zweckgerichtete Auswahl von Versuchsreihen zur Erfassung bzw. Katalogisierung der von einer Vielzahl von Parametern abhängenden Ergebnisse und insb. zur Prozeßoptimierung angegewandt und so eine erhebliche Minderung der Anzahl der durchzuführenden Versuche dadurch erreicht, daß ein Vermutungsbereich für eine Vielzahl von Parametern nicht mehr systematisch in einer Vielzahl von Versuchsreihen abgerastert wird, sondern, ausgehend von zufällig gewählten Punkten innerhalb

3

des Vermutungsbereichs sich abzeichnende Verbesserungen, mit stärkerem Gewicht in die nächste Versuchsgruppe eingehen, der zusätzlich jedoch wiederum gewisse gewillkürte oder Zufallsanteile beigeordnet werden.

Der erfindungsgemäße Einsatz von GA ist für die Optimierung beliebiger Systeme geeignet, für die eine Abhängigkeit zwischen den variierten Parametern und dem Optimierziel besteht. Einige Beispiele sind hier angegeben, die aber keinen Anspruch auf Vollständigkeit erheben. Systeme, bei denen eine Optimierung mit GA vorteilhaft sein könnte, sind:

Zusammensetzung chemischer Reaktionssysteme:

- Konzentration der Reaktanden
- Konzentration der Reaktanden und des Katalysators
- Art des Katalysators, Kombination und Katalysatoren
- Zusammensetzung des Katalysators
- Wahl oder Kombination von Lösungsmitteln

Reaktionsbedingungen chemischer Reaktionssysteme:

- Temperatur
- pH
- Druck
- Reaktionszeit
- Mischzeit
- Zeit für die Zugabe einzelner Reaktanden zum Reaktionssystem

Biologische Systeme:

- Zu den Parametern chemischer Systeme kommt noch hinzu: Zusammensetzung des Nährmediums
    mineralische Medien
    komplexe Medien
    Suplementierung komplexer Medien, mit einzelnen definierten Bestandteilen, z.B. Spurenelementen oder Vitaminen
- Begasung
- Umsetzungen mit Enzymen

Betriebsweise analytischer Systeme am Beispiel der HPLC:

- Mischungsverhältnis der Lösungsmittel
- Art des Gradienten
- Säulentemperatur
- Druck
- Fluß

Optimierung der Rezeptur für Farben, Aromen, Gerüche etc.

Die Anzahl der Versuche der primären Versuchsgruppe bzw. -reihe, welche die Zahl der Bitstrings gibt, wird zweckmäßigerweise zwischen 5 und 160, insbesondere zwischen 5 und 30 gewählt. Zwei Bitstrings pro Untersuchungsparameter sind angemessen.

Die Anzahl der Bits pro Parameter (Segment), durch welche die Feinheit der Rasterung bestimmt wird, kann von Parameter zu Parameter unterschiedlich gewählt werden und grundsätzlich beliebig hoch sein. Eine Bitzahl von 1 bis 10 pro Parameter (1 bis 1024 Unterteilungen des Vermutungsbereichs) erscheint im allgemeinen als ausreichend. Als "Rasterung" wird dabei die Anzahl der Unterteilungen (in codierter Form) verstanden.

Die Wahrscheinlichkeiten für Cross-over, Inversion und Mutation werden insbesondere in den Bereichen 0,25 - 1; 0 - 1 und 0 - 1 gewählt.

Zum Cross-over werden je zwei Bitstrings einander zufällig zugeordnet und ein oder mehrere beliebige Cross-over-Punkte festgesetzt.

Die Cross-over-Operation bei 4 Bitstrings mit Vorzugswichtung von II gestaltet sich für einen Cross-over-Punkt z.B. wie folgt:

```
I      0110|15        01100
II     1100|02        11001   Cross-over
                                       ⟶
II     11|0005        11011
IV     10|0112        10000
```

Wahrscheinlichkeit für Cross-over liegt zweckmäßigerweise zwischen 0,25 und 1, vorzugsweise zwischen 0,6 und 1.

Durch Inversion wird die Reihenfolge der einzelnen Bits im Bitstring vertauscht. Die Bedeutung der einzelnen Bits ändert sich dadurch nicht. Dies erreicht man durch eine Numerierung der einzelnen Bits, wie z.B.:

```
vor Inversion                      nach Inversion

Position:   12|3456|78             Position:   12|6543|78

A =         10|1110|11             A =         10|0111|11
```

(die Inversions-Punkte sind durch Striche dargestellt),

wodurch die Bedeutung eines Bits unabhängig von seiner Position wird. Inversion wirkt sich erst in den nächsten Generationen zusammen mit Cross-over aus. Durch diese Trennung von Position und Bedeutung eines Bits sind einige Optimierprobleme besser lösbar, z.B. und vereinfacht können bestimmte Parameterkombinationen durch Inversion im String räumlich näher positioniert und somit durch Cross-over schlechter zerstört werden.

Inversion ist also die Vertauschung der Reihenfolge der einzelnen Bits unter Beibehaltung ihrer Bedeutung, an einem oder zwischen mehreren zufälligen Punkten der Bitstrings. Die Wahrscheinlichkeit für Inversion liegt zwischen 0 und 1, vorzugsweise zwischen 0 und 0,2.

Mutation variiert den Bitstring-Satz durch punktuellen Austausch eines Bits und verhindert damit vorzeitige Konvergenz. D.h., mit einer bestimmten Wahrscheinlichkeit zwischen 0 und 1 (vorzugsweise 0,0001 - 0,01) wird im Bitstring eine Null gegen eine Eins bzw. umgekehrt ausgetauscht.

Die entstandene neue Population ersetzt die alte Generation mit einer Wahrscheinlichkeit von G = 0,3 bis 1,0. Ein Anteil von N * (1 - G) der alten Population kommt unverändert in die neue Population. G liegt vorzugsweise zwischen 0,8 und 1,0.

Anschließend erfolgt segmentweise die Transformation in die Realwerte der Parameter für die folgende Versuchsgruppe.

Zweckmäßierweise erfolgt erfindungsgemäß eine Umwandlung in zu maximierende Funktionen: In normalen Operations Research Arbeiten wandelt man ein Minimierungs- in ein Maximierungsproblem durch die Multiplikation mit (-1) um. Dies ist bei Genetischen Algorithmen nicht anwendbar. Bei Genetischen Algorithmen üblich ist folgende Transformation zur Überführung in ein Maximierungsproblem:

$$f(x) = C_{max} - g(x) \text{ für } g(x) < C_{max} \qquad (1)$$
$$f(x) = 0 \text{ andernfalls.}$$

$C_{max}$ ist ein Koeffizient, der beispielsweise das größte g(x) der Population oder der k letzten Populationen sein kann.

Die Transformation in positive Werte wird durch

$$f(x) = u(x) + C_{min} \text{ für } u(x) + C_{min} > 0 \qquad (2)$$
$$f(x) = 0 \text{ andernfalls}$$

realisiert. Auch hier kann $C_{min}$ frei gewählt werden, z.B. als schlechtester Wert der aktuellen oder mehrerer vorheriger Populationen. k liegt zwischen 0 und 7, wobei 0 konstant die 0 Generation, eins die aktuelle Generation und 2 - 6 die entsprechend zurückliegenden Generationen bedeutet. 7 heißt, daß die Umwandlung nicht vorgenommen wird. Vorzugsweise liegt k zwischen 1 und 3. Zweckmäßigerweise ist $C_{min}$ der kleinste u(x) der aktuellen oder einer vorangehenden Population.

**Skalierung:** Die Güten werden linear skaliert. Mit f' als transformierter und f als ursprünglicher Güte ist

$$f' = af + b. \qquad (3)$$

a und b müssen so gewählt werden, daß die durchschnittlichen Güten von f und f' identisch sind, so daß durchschnittliche Mitglieder weiterhin genau einen nachfolgenden Versuch erzeugen. Desweiteren kann durch den eingeführten Koeffizienten $C_{mult}$ die Anzahl der "Nachkommen" des Besten gesteuert werden, wenn gilt $f'_{max} = C_{mult} * f_{avg}$. ($C_{mult}$ liegt bei kleinen Populationen im Bereich zwischen 1,2 und 10, insbesondere zwischen 1,2 und 2).

**Selektion:** ("survival of the fittest") und anschließende Reproduktion dienen zur Vervielfältigung von Individuen (Parametersätzen), die eine überdurchschnittliche Anpassung (Güte) aufweisen. Die Wahrscheinlichkeit, "Nachkommen" für die nächste "Generation" zu zeugen, ist proportional zum Verhältnis der Güte des einzelnen Individuums zum Durchschnitt aller Güten der aktuellen Population (Bewertungszahl r).

Die verwendete Selektionsstrategie wurde von James E. Baker, "Reducing bias and inefficiency in the selection algorithm" in: Genetic Algorithms and their Applications: Proceedings of the Second International Conference on Genetic Algorithms, S. 14-21, Lawrence Erlbaum Associates, Publishers Hillsdale, New Jersey 1987, vorgeschlagen:

1 Ermittlung einer Zufallszahl x; ($0 \leq x \leq 1$)
2 Aufsummieren der Bewertungszahl r jedes Individuums bis: $\Sigma r > x$
3 Auswahl dieses Bitstrings
4 $x = x + 1$
5 Wenn $\Sigma r > x$ dann nochmalige Auswahl des Individuums, sonst weiter bei 1
6 Wiederholung, bis die gewünschte Anzahl an Bitstrings erreicht ist.

Eine Optimierungsstrategie nach Pareto "Cours d'Economie Politique", Rouge, Lausanne (1896) zur gleichzeitigen Optimierung mehrerer Zielgrößen wird dadurch realisiert, daß die Population in Untergruppen, gleicher oder unterschiedlicher Größe, entsprechend der Anzahl der Ziele aufgeteilt wird und für jede Gruppe ein Selektionsverfahren durchgeführt wird, wobei jedoch über alle Mitglieder der Population selektiert wird.

Die so ermittelte intermediäre Population wird nun den genetischen Operatoren unterworfen.

Es folgen Beispiele für die Durchführung der Erfindung:

Beispiel 1:

Experimentelle Ermittlung der Mediumszusammensetzung (5 Komponenten) für maximale Zellmassekonzentration beim aeroben Totalabbau von Benzoat mit *Pseudomonas putida*

Das aerobe Bakterium *Pseudomonas putida* ist in der Lage, in Wasser gelöstes Benzoat zusammen mit Sauerstoff zu Zellmasse, Energie, Kohlendioxyd und Wasser umzusetzen. Die vollständige Umsetzung von Benzoat kann jedoch nur erfolgen, wenn alle anderen zum Zellmasseaufbau notwendigen Substanzen (Hauptelemente wie Stickstoff, Phosphor, Schwefel..., Spurenelemente wie Mg, Mn, Zn... sowie eventuell Wuchsstoffe wie Aminosäuren, Vitamine...) in der wäßrigen Lösung ('Nährmedium') in ausreichendem Maße vorhanden sind.

Die optimale Zusammensetzung dieses organismusspezifischen Nährmediums soll (in Bezug auf die umzusetzende Benzoatmenge) für 5 ausgewählte Mediumskomponenten (siehe Tab. 1) experimentell ermittelt werden. Die anderen notwendigen Mediumskomponenten werden in diesem Beispiel konstant gehalten (10 mmol/l Benzoat, 28 mmol/l $NH_4Cl$, 204 $\mu$mol/l $CaCl_2$, 1.5 mmol/l $H_3PO_4$, 0.534 $\mu$mol/l $CoSO_4$, 1.2 $\mu$mol/l $CuSO_4$, 36.4 $\mu$mol/l $H_3BO_4$, 1.24 $\mu$mol/l $Na_2MoO_4$).

Die experimentellen Untersuchungen (Versuche) werden in 100 ml Schüttelkolben mit 10 ml Reaktionsvolumen im Batch-Ansatz durchgeführt ('Optimierung der Start-Mediumszusammensetzung'). Die Reaktionsbedingungen werden fest vorgegeben:

| - Animpfmenge (gewaschene Zellen aus Lagerung bei -20 ° C):exponentieller Wachstumsphase, Lagerung bei -20 ° C): | 1 % |
| - Temperatur : | 37 ° C |
| - Schüttelfrequenz: | 150 rpm |
| - Prozeßzeit: | 16.5 h |
| - Meßgröße: | $OD_{660}$ |
| (Optische Dichte bei einer Wellenlänge von 660 nm, 10 mm Küvette: als Maß für die Zellmassenkonzentration) | |

Die zu variierenden Konzentrationsbereiche ('Abtastbereiche') der einzelnen Mediumskomponenten werden so gewählt, daß die Konzentration jeder Mediumskomponente um etwa 1 Zehnerpotenz verringert und etwa 1 Zehnerpotenz gegenüber dem 'Ausgangsmedium' erhöht werden kann (siehe Tab. 1). Als Ausgangsmedium stand in diesem Fall ein der Literatur entnommenes Medium zur Verfügung.

| Komponente | Stamm-lösung | Medium Minimal | Medium Ausgang | Medium Maximal | Einheit |
|---|---|---|---|---|---|
| KCl | 100 | 0 | 1.5 | 10 | mmol/l |
| $MgSO_4$ | 40.6 | 0 | 0.609 | 4.06 | mmol/l |
| $FeSO_4$ | 719 | 0 | 10.8 | 71.9 | µmol/l |
| $MnSO_4$ | 1180 | 0 | 17.7 | 118 | µmol/l |
| $ZnSO_4$ | 278 | 0 | 4.17 | 27.8 | µmol/l |

Tab. 1: Konzentrationen, mögliche Konzentrationsbereiche der zu variierenden Mediumskomponenten

Die Rasterung, also die Anzahl der möglichen Konzentrationsstufen im jeweiligen Abtastbereich (in der statistischen Versuchsplanung auch als 'Niveaus' bezeichnet) wird mit 200 + 1(Nullwert) = 201 vorgegeben, um die Optimalkonzentration jeder Mediumskomponente möglichst genau ermitteln zu können.

Die technische Realisierung dieser Rasterung erfolgt durch Zugabe einer Stammlösung der jeweiligen Mediumskomponente. Da die verwendeten Pipetten nur eine minimale Auflösung von 5 µl gestatten, ergibt sich als maximal dosierbares Volumen je Mediumskomponente 1 000 µl (Minimalvolumen: 0 µl). Die optimalen Volumina der einzusetzenden Stammlösungen ( = Konzentrationen der Mediumskomponenten) sollen experimentell ermittelt werden:

Die Codierung der Volumina der zu variierenden Stammlösungen ('Parameter') erfolgt als bitstrings. Um die Rasterung von 201 realisieren zu können, sind 8 bit pro Parameter notwendig ($2^8$ = 256). Damit ergibt sich eine string-Länge von 40 bit, um eine komplette 'Versuchsvorschrift' (Mediumszusammensetzung, Mengen der einzusetzenden Stammlösungen) zu codieren.

Die Versuche werden im Parallelansatz ('Generation') durchgeführt. Die Anzahl der parallel durchzuführenden Versuche wird auf 12 festgesetzt, das bedeutet 2 Parallelversuche je Parameter (5 Stammlösungen) und je Ziel (maximale Zellmassenkonzentration gemessen als OD).

Die Parameterwerte der 0. Generation (Start-Generation) werden bis auf die Startmediums-Zusammensetzung in Binärcode-Darstellung durch Zufall ermittelt. Die Decodierung dieser Binärcode-Darstellung in das gewohnte decadische Zahlensystem erfolgt wie üblich:

Beispiel für einen Parameter :

$00010011 = 0*2^7 + 0*2^6 + 0*2^5 + 1*2^4 + 0*2^3 + 0*2^2 + 1*2^1 + 1*2^0 = 19$
Die Umrechnung der decodierten Zahl in den Parameterwert (Menge der einzusetzenden Stammlösung, µl)

erfolgt wie angegeben:

Parameterwert =
numerisches Ergebnis * Abtastbereich / maximalen
decodierten bit-Wert + Parameter-Minimalwert

analog obigem Beispiel:

Parameterwert = 19 * (1 000 - 0) $\mu$l / 256 + 0 = 74.2 $\mu$l,
gerundet 75 $\mu$l.

Die 12 Versuche der 0. Generation werden unter standardisierten Bedingungen parallel durchgeführt und die Zielgröße (OD) gemessen (Mengen der eingesetzten Stammlösungen und Meßergebnisse der Generation 0 siehe Tab. 2).

Zur Auswertung der in der 0. Generation erhaltenen Ergebnisse wird eine Transformation (Umwandlung in eine zu maximierende Funktion, Vermeidung von negativen Ergebnissen) und eine Skalierung der Meßwerte vorgenommen:

Da es sich in diesem Beispiel bereits um ein Maximierungsproblem handelt, erfolgt zur Transformation lediglich die Addition des niedrigsten Meßwertes ('$C_{min}$' = 0.05) zu allen Meßergebnissen (siehe Tab. 2).

Zur Skalierung der Meßergebnisse werden die Konstanten a und b der Skalierungsgleichung:

$$f' = a * f + b$$

ermittelt (f', f skalierte, bzw. transformierte 'Güte der Zielfunktion', hier OD):

$$a = f_{avg} * (C_{mult} - 1) / (f_{max} - f_{avg})$$
$$b = f_{avg} * (1 - a)$$

$f_{avg}$ (= 0.242) ist der arithmetische Mittelwert, $f_{max}$ (= 0.506) der Maximalwert der transformierten 'Güte der Zielfunktion' (OD transformiert).

Die Steuergröße $C_{mult}$ als Maß für die Verstärkung des besten Ergebnisses wird in diesem Beispiel auf $C_{mult}$ = 2 gesetzt. Damit ergibt sich für die Konstanten der Skalierungsgleichung:

a = 0.917 b = 0.02

Mit Hilfe der Skalierungsgleichung werden die transformierten Meßergebnisse skaliert ( siehe Tab. 2).

Die Berechnung der Bewertungszahl r, als Basis zur Durchführung der 'Selektion' der bisher besten Medienzusammensetzungen zur weiteren Bearbeitung mit den 'genetischen Operatoren' Crossover und Mutation, erfolgt nach:

$$r = f' / f_{avg} * 11/12$$

Die Multiplikation mit 11/12 erfolgt, da der bit-string (die Versuchsvorschrift) mit dem besten Ergebnis unverändert in die nächste Generation (Versuchsreihe) übernommen werden soll, und daher ein Platz weniger für die Selektion vorhanden ist (Dies ist in diesem Beispiel Versuchsvorschrift Nr. 9, siehe Tab. 2).

| Nr | KCl $\mu l$ | MgSO$_4$ $\mu l$ | FeSO$_4$ $\mu l$ | MnSO$_4$ $\mu l$ | ZnSO$_4$ $\mu l$ | OD - | f - | f' - | r - |
|---|---|---|---|---|---|---|---|---|---|
| 1 | 150 | 150 | 150 | 150 | 150 | 0.066 | 0.116 | 0.126 | 0.477 |
| 2 | 125 | 800 | 785 | 955 | 100 | 0.337 | 0.387 | 0.375 | 1.42 |
| 3 | 695 | 105 | 445 | 925 | 530 | 0.142 | 0.192 | 0.196 | 0.742 |
| 4 | 845 | 510 | 265 | 5 | 315 | 0.144 | 0.194 | 0.198 | 0.75 |
| 5 | 440 | 635 | 205 | 595 | 285 | 0.147 | 0.197 | 0.201 | 0.761 |
| 6 | 625 | 505 | 580 | 515 | 255 | 0.291 | 0.341 | 0.333 | 1.261 |
| 7 | 600 | 895 | 60 | 525 | 960 | 0.175 | 0.225 | 0.226 | 0.856 |
| 8 | 515 | 705 | 350 | 405 | 635 | 0.242 | 0.292 | 0.288 | 1.091 |
| 9 | 955 | 610 | 835 | 35 | 380 | 0.456 | 0.506 | 0.484 | 1.833 |
| 10 | 95 | 665 | 270 | 830 | 355 | 0.169 | 0.219 | 0.221 | 0.837 |
| 11 | 20 | 200 | 740 | 375 | 405 | 0.05 | 0.1 | 0.112 | 0.424 |
| 12 | 635 | 660 | 160 | 920 | 635 | 0.08 | 0.13 | 0.139 | 0.527 |

Tab. 2: Vorgelegte Mediumskomponenten, gemessene Güte OD, transformierte Güte f, skalierte Güte f', Bewertungszahl r der Generation 0.

Die Selektion erfolgt nach Ermittlung einer Zufallszahl x (in diesem Fall x = 0.63) wie in der Beschreibung angegeben, siehe Tab. 3):
Die Bewertungszahl von Versuch 1 ist 0.477. Da die Zufallszahl x größer als diese Bewertungszahl ist, wird diese Versuchsvorschrift (bit-string) nicht selektiert. Die Bewertungszahl von Versuch 2 ist 1.42, womit sich als Summe der bisherigen Bewertungszahlen 1.898 ergibt. Jetzt ist die Bewertungszahlsumme größer als die Zufallszahl, so daß Versuchsvorschrift 2 selektiert wird. Die Zufallszahl wird im folgenden Schritt um 1 auf 1.63 erhöht. Sie ist noch immer kleiner als die Bewertungszahlsumme, so daß Versuchsvorschrift 2 erneut selektiert wird. Eine erneute Erhöhung der Zufallszahl x um 1 auf 2.63 führt dazu , daß x jetzt größer ist als die Bewertungszahlsumme, so daß das Verfahren mit Versuch 3 weitergeführt wird u.s.w. (siehe Tabelle 3).

| Nr. | r | Summe(r) | x, x+1 | Selektion x<Summe(r)? | selektiert Nr. |
|---|---|---|---|---|---|
| 1 | 0.477 | 0.477 | 0.63 | nein | |
| 2 | 1.42 | 1.897 | 0.63 | ja | 2 |
| | | 1.897 | 1.63 | ja | 2 |
| | | 1.897 | 2.63 | nein | |
| 3 | 0.742 | 2.639 | 2.63 | ja | 3 |
| | | 2.639 | 3.63 | nein | |
| 4 | 0.75 | 3.389 | 3.63 | nein | |
| 5 | 0.761 | 4.150 | 3.63 | ja | 5 |
| | | 4.150 | 4.63 | nein | |
| 6 | 1.261 | 5.411 | 4.63 | ja | 6 |
| | | 5.411 | 5.63 | nein | |
| 7 | 0.856 | 6.267 | 5.63 | ja | 7 |
| | | 6.267 | 6.63 | nein | |
| 8 | 1.091 | 7.358 | 6.63 | ja | 8 |
| | | 7.358 | 7.63 | nein | |
| 9 | 1.833 | 9.191 | 7.63 | ja | 9 |
| | | 9.191 | 8.63 | ja | 9 |
| | | 9.191 | 9.63 | nein | |
| 10 | 0.837 | 10.028 | 9.63 | ja | 10 |
| | | 10.028 | 10.63 | nein | |
| 11 | 0.424 | 10.452 | 10.63 | nein | |
| 12 | 0.527 | 10.979 | 10.63 | ja | 12 |

Tab. 3: Selektion, Vorgehensweise bei der Selektion

Nach Abschluß der Selektion zeigt sich, daß in diesem Fall die Versuchsvorschriften 2 und 9 doppelt selektiert werden, während die Versuchsvorschriften 1, 4 und 11 nicht übernommen werden.

Die selektierten bit-strings werden danach jeweils paarweise einem crossover unterzogen. Die Auswahl der Paare erfolgt zufällig, die crossover-Wahrscheinlichkeit ist zu 95 % festgelegt worden. Die Durchführung des crossover ist z.B. für 2 beliebige bit-strings im folgenden dargestellt, wobei der zufällig gewählte crossover-Punkt durch * markiert ist:

```
11110001   10101*100   10110101   01111100   10101111

00011101   01010*111   00101110   10101001   01010111
```

neue bit-strings:

10

```
11110001   10101111   00101110   10101001   01010111

00011101   01010100   10110101   01111100   10101111
```

Nach der crossover Operation werden an den so neu generierten bit-strings Punktmutation durchgeführt, in dem eine 1 gegen eine 0 oder umgekehrt ausgetauscht wird. Die Mutation erfolgt zufällig mit einer Mutationswahrscheinlichkeit von 0.1 %. Am Beispiel des oben neu generierten bit-strings ist eine Mutation dargestellt, markiert durch eine eckige Klammer:

```
00011101   01010100   10110101   01[1]11100   10101111
```

neuer bit-string:

```
00011101   01010100   10110101   01[0]11100   10101111
```

Die so neu erzeugten bit-strings werden anschließend decodiert und die Versuche wie oben beschrieben durchgeführt (1. Generation). Dieser Zyklus wird in diesem Beispiel insgesamt 7 mal durchlaufen (Generation 0-6).

Die Einstellungen des Genetischen Algorithmus für alle Generationen sind im folgenden noch einmal zusammengefaßt:

| Steuergröße zur Skalierung: | $C_{mult}$ | = 2 |
|---|---|---|
| crossover Wahrscheinlichkeit: | | = 95 % |
| Mutations Wahrscheinlichkeit: | | = 0.1 % |
| Inversions Wahrscheinlichkeit: | | = 0.0 % |
| Wahrscheinlichkeit für neue Generation: | | = 100 % |

Die Crossover-Wahrscheinlichkeit wird wesentlich höher gewichtet, als die Mutations Wahrscheinlichkeit, weil durch Kombination der Parameter schneller das Optimum gefunden wird. Inversion verbessert in diesem Beispiel die Suche nicht und wird daher zu 0 gesetzt. Die Wahrscheinlichkeit für eine neue Generation (Austausch der bit-string Folge) wird zu 100 % gesetzt, da so der Abtastbereich maximal abgesucht wird.

Ergebnis: Durch Einsatz des Genetischen Algorithmus zur experimentellen Ermittlung der Startkonzentration von 5 ausgewählten Mediumskomponenten zur Maximierung der Zellmassenkonzentration beim aeroben Totalabbau von Benzoat mit Pseudomonas putida konnte mit insgesamt 84 Versuchen die Zellmassenkonzentration um den Faktor 9.4 gesteigert werden ($OD_{Startmedium}$ 0.045, $OD_{Optimalmedium}$ 0.424).

Die Zusammensetzung von Start- und Optimalmedium ist in Tabelle 4 dargestellt:

| Komponente | Ausgangsmedium | Optimalmedium | Einheit |
|---|---|---|---|
| KCl | 1.5 | 8.3 | mmol/l |
| $MgSO_4$ | 0.609 | 3.49 | mmol/l |
| $FeSO_4$ | 10.8 | 60.1 | $\mu mol/l$ |
| $MnSO_4$ | 17.7 | 4.14 | $\mu mol/l$ |
| $ZnSO_4$ | 4.17 | 0.278 | $\mu mol/l$ |

Tab. 4: Konzentrationen Ausgangs- und Optimalmedium

Der Vergleich mit herkömmlich zur Mediumsoptimierung eingesetzten Techniken der statistischen Versuchsplanung zeigt die Überlegenheit des erfindungsgemäßen Verfahrens zur experimentellen Ermittlung der Parameter für optimale Ergebnisse:

S. Bloor und R.R. England (Enzyme Microb. Technol. 13 (1991) 76-81) setzten zum Beispiel 'factorial experimental design' ein, um ebenfalls die Konzentration von 5 Mediumskomponenten zu optimieren. Sie führten 16 Versuche durch, um lediglich 2 Konzentrationsstufen (Niveaus) jeder Mediumskomponente zu untersuchen (+/- 50 %-Wert des Ausgangsmediums). Damit waren bei 5 Parametern 8 Versuche je Konzentrationsstufe erforderlich. Mit dem hier entwickelten Verfahren mit weitaus höherer Auflösung (201 Konzentrationsstufen oder Niveaus) sind dagegen nur 0,4 Versuche je Konzentrationsstufe erforderlich.

Die erforderliche Anzahl der durchzuführenden experimentellen Versuche kann also, wie dieses Beispiel zeigt, gegenüber Techniken der bekannten statistischen Versuchsplanung um den Faktor 20 reduziert werden.

Beispiel 2:

Experimentelle Ermittlung der Mediumszusammensetzung (12 Komponenten) für maximale Zellmassekonzentration beim aeroben Totalabbau von Benzoat mit *Pseudomonas putida*

Ziel einer 2. Optimierung war es, neben der Maximierung der Zellmassenkonzentration durch Optimierung der Startkonzentration von 12 Nährsalzen und Spurenelementen gleichzeitig alle 12 eingesetzten Mediumskomponenten in einer Versuchsabfolge zu minimieren. Es sollten also gleichzeitig 13 Optimierziele verfolgt werden, daher wurde nach der von Pareto konzipierten Vektoroptimierung vorgegangen.

Unter denselben experimentellen Bedingungen wie zuvor wird eine Optimierung durchgeführt, lediglich die Rasterung wird von 201 auf 101 Konzentrationsniveaus je Mediumskomponente verringert.

Damit sind noch 7 bit pro Parameter erforderlich ($2^7$ = 128). Die string-Länge ergibt sich bei 12 Parametern zu 84 bit.

Die Konzentration der eingesetzten Stammlösungen, sowie der abgedeckte Konzentrationsbereich sind in Tabelle 5 zusammengefaßt.

Die Anzahl der parallel durchzuführenden Versuche je Generation wird auf 28 festgesetzt, das bedeutet 2 Versuche je zu minimierende Mediumskomponente und 4 Versuche für das Ziel Zellmassekonzentration, das somit ein etwas stärkeres Gewicht erhält.

| Komponente | Stamm-lösung | Medium Minimal | Medium Ausgang | Medium Maximal | Einheit |
|---|---|---|---|---|---|
| $NH_4Cl$ | 3.74 | · 0 | 0.028 | 0.187 | mol/l |
| $H_3PO_4$ | 200 | 0 | 1.5 | 10 | mmol/l |
| KCl | 200 | 0 | 1.5 | 10 | mmol/l |
| $MgSO_4$ | 81.1 | 0 | 0.609 | 4.06 | mmol/l |
| $FeSO_4$ | 1440 | 0 | 10.8 | 71.9 | µmol/l |
| $MnSO_4$ | 1180 | 0 | 17.7 | 59.2 | µmol/l |
| $ZnSO_4$ | 278 | 0 | 4.17 | 13.9 | µmol/l |
| $CuSO_4$ | 160 | 0 | 1.2 | 8.01 | µmol/l |
| $CoSO_4$ | 71.1 | 0 | 0.534 | 3.56 | µmol/l |
| $CaCl2$ | 27.2 | 0 | 0.204 | 1.36 | mmol/l |
| $H_3BO_4$ | 4.85 | 0 | 0.0364 | 0.243 | mmol/l |
| $Na_2MoO_4$ | 165 | 0 | 1.24 | 8.27 | µmol/l |

Tab. 5: Konzentrationen, mögliche Konzentrationsbereiche der zu variierenden Mediumskomponenten

Zur Auswertung der Experimente analog zum Beispiel 1 muß für die zu minimierenden Parameter eine Umwandlung in zu maximierende Werte vorgenommen werden, das heißt die Parameterwerte (g) jeder einzelnen Mediumskomponente werden von ihrem jeweiligen maximalen Wert $C_{max}$ substrahiert:

f = $C_{max}$ - g für g < $C_{max}$;
f = 0 für g > $C_{max}$.

Die Transformation und Skalierung erfolgt wie in Beispiel 1 gezeigt. Bei der Ermittlung der Bewertungszahl r muß für jedes Ziel eine Bewertungszahl r ermittelt werden (13 Bewertungszahlen). Bei der Selektion muß dann entsprechend die bit string Folge in eine Gruppe mit 4 bit strings (Bewertungszahl OD) und 12 Gruppen mit 2 bit strings (Bewertungszahlen je Mediumskomponente) unterteilt werden.

Diese Optimierung wurde analog dem Beispiel 1 bis zur 7. Generation durchgeführt, der Zyklus also insgesamt 8 mal durchlaufen (Generation 0-7).

Ergebnis: Durch Einsatz des Genetischen Algorithmus zur experimentellen Ermittlung der Startkonzentration von 12 ausgewählten Mediumskomponenten zur Maximierung der Zellmassenkonzentration und gleichzeitiger Minimierung der eingesetzten Mediumskomponenten beim aeroben Totalabbau von Benzoat mit Pseudomonas putida konnte mit insgesamt 224 Versuchen die Zellmassenkonzentration um den Faktor 28 gesteigert werden ($OD_{Startmedium}$ 0.045, $OD_{Optimalmedium}$ 1.272).

Die Zusammensetzung von Start- und Optimalmedium ist in Tabelle 6 dargestellt.

| Komponente | Ausgangsmedium | Optimalmedium | Einheit |
|---|---|---|---|
| (Benzoat | 10 | 10 | mmol/l) |
| $NH_4Cl$ | 28 | 22.4 | mmol/l |
| $H_3PO_4$ | 1.5 | 7.9 | mmol/l |
| KCl | 1.5 | 8.3 | mmol/l |
| $MgSO_4$ | 0.609 | 3.49 | mmol/l |
| $FeSO_4$ | 10.8 | 60.1 | $\mu$mol/l |
| $MnSO_4$ | 17.7 | 4.14 | $\mu$mol/l |
| $ZnSO_4$ | 4.17 | 0.278 | $\mu$mol/l |
| $CuSO_4$ | 1.2 | 0.881 | $\mu$mol/l |
| $CoSO_4$ | 0.534 | 1.32 | $\mu$mol/l |
| $CaCl_2$ | 204 | 639 | $\mu$mol/l |
| $H_3BO_4$ | 36.4 | 72.8 | $\mu$mol/l |
| $Na_2MoO_4$ | 1.24 | 1.16 | $\mu$mol/l |

Tab. 6: Konzentrationen Ausgangs- und Optimalmedium

Auch hier zeigt ein Vergleich von herkömmlich zur Mediumsoptimierung eingesetzten Techniken der statistischen Versuchsplanung die Überlegenheit des entwickelten Verfahrens zur experimentellen Ermittlung der Parameter für optimale Ergebnisse:
Roseiro et al. (Process Biochemistry 27 (1992) 167-175) setzten zum Beispiel 'factorial experimental design' nach Plackett and Burman ein, um die Konzentration von 11 Mediumskomponenten zu optimieren. Sie führten 28 Versuche durch, um lediglich 2 Konzentrationsstufen (Niveaus) jeder Mediumskomponente zu untersuchen (+/- 20 %-Wert des Ausgangsmediums). Damit waren bei 11 Parametern 14 Versuche je Konzentrationsstufe erforderlich.

Mit dem hier entwickelten Verfahren mit weitaus höherer Auflösung (101 Konzentrationsstufen oder Niveaus) sind dagegen nur 2 Versuche je Konzentrationsstufe erforderlich. Dabei wird jedoch nicht nur 1 Ziel wie bei Roseiro et al. verfolgt, sondern gleichzeitig 13 Ziele.

Weitere aktuelle Beispiele für die bisher eingesetzte Technik 'statistische Versuchsplanung' zur Optimierung der Startmediums-Zusammensetzung:
R. G. Silveira et al. : J. of Ferment. and Bioeng. 72, 1 (1991), 20-25.
    R. Banerjee, B.C. Bhattacharyya: Biotechnol. and Bioeng. 41 (1993), 67-71.
    P. V. Rao et al.: Process Biochemistry 28 (1993), 391-395.

**Patentansprüche**

1.  Iteratives Verfahren zur experimentellen Ermittelung der Parameterwerte für ein optimales Prozeßergebnis innerhalb von vorgegebenen Bereichsgrenzen für eine Vielzahl von Parametern nicht bekannter Interdependanz und Ergebnis-Relevanz durch Aufeinanderfolge von Versuchsreihen mit jeweils innerhalb der Bereichsgrenzen abgewandelten Parametervorgaben,
    **dadurch gekennzeichnet,**
    daß die Parametervorgaben, jeder Versuchsreihe - ausgehend von den Ergebniswerten einer ersten, durch Zufallswerte der Parameter festgelegten Versuchsreihe - durch Anwendung einer dem genetischen Evolutions-Algorithmus (nach Holland) folgenden Optimierungsstrategie unter Zugrundelegung der Ergebniswerte der jeweils vorangehenden Versuchsreihe festgelert werden.

**2.** Verfahren nach Anspruch 1,
**dadurch gekennzeichnet,**
daß

Ia) eine der beliebig wählbaren Anzahl der Versuche der primären Versuchs reihe entsprechende Anzahl von Bitstrings mit einer der Anzahl der Parameter entsprechenden Anzahl von Segmenten mit jeweils der gewünschten Rasterung entsprechenden Bitzahl durch Zufallskodierung gebildet wird,

Ib) deren numerisches Ergebnis multipliziert mit dem Quotienten aus Abtastbereich durch maximalen decodierten Bitwert zum Parameter-Minimalwert addiert und ggf. aufgerundet je Segment die Realwerte der einzelnen Versuchsparameter der primären Versuchsreihe ergibt, daß

II) die Versuche der primären Versuchsreihe mit den so ermittelten Parametern durchgeführt werden unter Erzielung einer Gruppe von Ergebnissen und daß

III) aus den erhaltenen Ergebnissen der Ergebnismittelwert gebildet wird, der einen Wichtungsschlüssel (aus Einzelergebnis der Versuchsreihe / Ergebnismittelwert der Versuchsreihe) liefert für die Zufallsauswahl einer intermediären Bitstring-Folge, die nacheinander mit abgestufter Wahrscheinlichkeit einem Cross-over (a), der Inversion (b) und der Mutation (c) unterworfen wird, wobei

a) innerhalb der Bitstring-Folge eine Zufallspaarung über alle Bitstrings vorgenommen wird und bei den so gebildeten Bitstringpaaren ein Austausch der einer zufällig gewählten Position folgenden Bits untereinander vorgenommen wird, worauf

b) hinter einer oder zwischen mehreren zufällen Positionen der mit gegenüber (a) mit geminderter Wahrscheinlichkeit ausgewählten Bitstrings eine Umkehr der Bit-Reihenfolge unter Beibehaltung ihrer Parameterzugehörigkeit und Platznummer stattfindet und schhließlich

c) mit wiederum verminderter Wahrscheinlichkeit ein Zufallsaustausch einzelner Bits vorgesehen wird,

daß die auf diese Weise erhaltene Bitstringfolge der anschließenden Versuchsreihe gemäß Ib in Realwerte umgesetzt wird, wobei ggf. der gemäß Wichtung beste Bitstring unverändert in die nächste Bitstringfolge eingereiht wird; und daß nach Durchführung der Versuche entsprechend der so festgelegten Versuchsvorschriften ggf. wiederkehrend erneut wie unter III verfahren wird.

**3.** Verfahren nach Anspruch 2,
**dadurch gekennzeichnet,**
daß die primäre Versuchsreihe 5 bis 30 Versuche umfaßt.

**4.** Verfahren nach Anspruch 2,
**dadurch gekennzeichnet,**
daß pro Parameter zwei Bitstrings vorgesehen werden.

**5.** Verfahren nach Anspruch 2,
**dadurch gekennzeichnet,**
daß die Zahl der Bitstrings je Versuchsreihe innerhalb der Aufeinanderfolge von Versuchsreihen konstant bleibt.

**6.** Verfahren nach einem der vorangehenden Ansprüche,
**dadurch gekennzeichnet,**
daß die Wahrscheinlichkeiten für Cross-over (a); Inversion (b) und Mutation (c) zwischen 0,25 und 1; 0 und 1; sowie 0 und 1 gewählt werden.

**7.** Verfahren nach Anspruch 2,
**dadurch gekennzeichnet,**
daß in Stufe III die erhaltenen Ergebnisse im Falle eines Minimierungsproblems in ein Maximierungsproblem nach

$f(x) = C_{max} - g(x)$ für $g(x) < C_{max}$
$f(x) = 0$ andernfalls,

umgewandelt werden,
und negative Gütewerte in positive Gütewerte nach

$$f(x) = u(x) + C_{min} \text{ für } u(x) + C_{min} > 0$$
$$f(x) = 0 \text{ andernfalls}$$

transformiert werden,

so skaliert werden, daß die Ergebnisse so gestaucht oder gestreckt werden, daß ein durchschnittliches Ergebnis seinen Bewertungsfaktor beibehält und daß der Bewertungsfaktor einen maximalen Wert nicht überschreitet, mit

$$f' = a^*f + b$$
$$f'_{max} = C_{mult}{}^*f_{avg} \, ,$$

die Wahrscheinlichkeit für Cross-over zwischen 0,6 und 1 , vorzugsweise 0,9 und 1 liegt, die Wahrscheinlichkeit für Inversion vorzugsweise 0,0 beträgt, die Wahrscheinlichkeit für Mutation zwischen 0,0001 und 0,1, vorzugsweise zwischen 0,001 und 0,05 liegt,

zusätzlich zum Selektionsverfahren der Beste eines Zieles in die nächste Generation übernommen wird, und

die Population in Untergruppen, gleicher oder unterschiedlicher Größe, entsprechend der Anzahl der Ziele aufgeteilt wird.

8. Verfahren nach Anspruch 7,
   **dadurch gekennzeichnet,**
   daß $C_{max}$ das größte $g(x)$ der aktuellen Population oder einer vorhergehenden Population ist,
   $C_{min}$ der kleinste $u(x)$ der aktuellen Population oder einer vorhergehenden Population ist, und
   $C_{mult}$ zwischen 1,2 und 10 liegt.

9. Verfahren nach Anspruch 7,
   **dadurch gekennzeichnet,**
   daß bei der Ermittlung der intermediären Bitstring-Folge für jedes Ziel, für jeden Bitstring ein Bewertungsfaktor ermittelt und für jedes Ziel ein Selektionsverfahren folgendermaßen stattfindet,
   1. Ermittlung einer Zufallszahl $x$; $(0 \leq x \leq 1)$
   2. Aufsummieren der Bewertungszahlen $r$ jedes Individuums bis Summe $(r) > x$;
   3. Auswahl dieses Bitstrings;
   4. $x = x + 1$;
   5. Wenn Summe $(r) > x$ nochmalige Auswahl dieses Bit-Strings, zurück zu 4, sonst zu 1;
   6. Wiederholung, bis die gewünschte Anzahl an neuen Bitstrings erreicht ist,
   wonach anschließend Cross-over, Inversion und Mutation erfolgen.